## Europäisches Patentamt

## European Patent Office

## Office européen des brevets

(11) Veröffentlichungsnummer: **0 054 673** A1

# EUROPÄISCHE PATENTANMELDUNG

(21) Anmeldenummer: **81108667.7**

(22) Anmeldetag: **22.10.81**

(51) Int. Cl.³: **G 01 N 25/04,** G 01 N 33/26, G 01 R 31/12

(30) Priorität: **24.12.80 DE 3049071**

(43) Veröffentlichungstag der Anmeldung: **30.06.82**
**Patentblatt 82/26**

(84) Benannte Vertragsstaaten: **CH FR GB IT LI NL SE**

(71) Anmelder: **Philips Kommunikations Industrie AG, Thurn-und-Taxis-Strasse 10, D-8500 Nürnberg 10 (DE)**

(72) Erfinder: **Völker, Wolfgang, Otto-Han-Strasse 2, D-3548 Arolsen (DE)**
Erfinder: **Finke, Hartmut, Dresdener Strasse 5, D-3548 Arolsen (DE)**
Erfinder: **Orth, Meinolf, Hinter den Höfen 42, D-3539 Udorf (DE)**

(54) Verfahren und Vorrichtung zum Prüfen eines Lackdrahtes auf seine Temperaturbeständigkeit (Schmorfestigkeit).

(57) Die Erfindung betrifft ein Verfahren und eine Vorrichtung zum Prüfen eines Lackdrahtes auf Hitze- bzw. Hitzedauerbeständigkeit durch dessen Erhitzen bis zum Verschmoren seiner Isolierung und Feststellung der hierbei erreichten Temperatur bzw. Zeitdauer. Sie löst die Aufgabe, den Zeit- bzw. Energieaufwand einer solchen Lackdraht-Qualitätsprüfung zu verringern.

Die Lösung besteht im wesentlichen in der gleichzeitigen Wickelanordnung von zwei Probelängen des zu prüfenden Lackdrahtes in einander gegenseitig berührenden Windungen auf einem gemeinsamen Träger und dem temperaturgeregelten Durchfluß eines Heizstromes durch die Windungen der einen Probelänge und einer Prüfspannung durch jene der anderen Probelänge, derart, daß der Heizstrom bei Verschmoren der Isolierung abgeschaltet wird.

Verfahren und Vorrichtung zum Prüfen eines Lackdrahtes auf
seine Temperaturbeständigkeit (Schmorfestigkeit)

Die Erfindung betrifft ein Verfahren zum Prüfen eines Lackdrahtes auf Hitze- bzw. Hitzedauer-Beständigkeit, nach dem
Oberbegriff des Anspruchs 1 sowie auf eine hierzu geeignete
Vorrichtung. Sie findet besonders Anwendung zur Qualitätsbestimmung von lackisolierten Wickel- oder Schaltdrähten, die
hohen Betriebstemperaturen ausgesetzt sind. Solche Betriebsbedingungen treten beispielsweise bei Hochleistungstransformatoren, hochbelasteten E-Motoren, beispielsweise für den
Scheibenwischerantrieb in Kraftfahrzeugen sowie bei für die
Verdrahtung von Vorschaltgeräten für Leuchtstoffröhren eingesetzten lackisolierten Schaltdrähten, die im Betrieb ebenfalls
sehr hohen Temperaturen ausgesetzt sind. Daraus ergibt sich
die Notwendigkeit, Proben von für derartige Anwendungen bestimmten Lackdrähten auf ihre Schmorfestigkeit, d.h. maximale
Hitzebeständigkeit bzw. Hitzedauerbeständigkeit zu prüfen.

Derartige Schmortests wurden bislang in einem äußerlich beheizbaren Ofen, genannt Heizschrank, mit in den Boden oder die
Wandung des wärmeisolierten Schrankes eingebauten elektrischen
Heizelementen durchgeführt. Dieser mußte hierzu insgesamt auf
die erforderliche hohe Temperatur von etwa 300 bis 400 °C erhitzt werden, um festzustellen, ob die zuvor eingelegten Probelängen des zu prüfenden Lackdrahtes einer solchen Temperatur standhalten können bis die Isolierung verschmort, oder um bei
etwas geringerer Temperatur die Zeitdauer bis zum Verschmoren
der Lackisolierung festzustellen. Dies erfolgte wegen der
langen Heizdauer bis zum Erreichen der hohen kritischen Temperatur, z.B. 400 °C, und des hierzu erforderlichen hohen Heizkostenaufwandes sowie wegen der entsprechend langen Rückkühlzeiten, welche notwendig waren, um den Heizschrank auf eine
geringere Schmortesttemperatur für andere Drahtproben abzukühlen, nur in Ausnahmefällen, etwa mit einem Probestück im Rahmen der Ausführung eines bestimmten Lackdraht-Fertigungsauftrages.

Der Erfindung liegt die Aufgabe zugrunde, den Schmortest soweit
zu vereinfachen, daß er mit geringem Aufwand kontinuierlich
zur Qualitätskontrolle, sowohl im Fertigungswerk als auch beim
Abnehmer solcher hochtemperaturbeständiger Lackdrähte durchgeführt werden kann. Die Lösung dieser Aufgabe ist mit der im
Anspruch 1 gekennzeichneten Erfindung angegeben.

Wesentlich ist bei diesem neuen Schmortest, die Verwendung
eines Teiles der Probelängen des zu prüfenden Lackdrahtes
selbst als Heizleiter, wobei die jeweilige Schmortemperatur
sehr schnell und mit geringem Energieaufwand erreicht wird,
sowie eine temperaturabhängige Regelung des durch diesen Teil
der in parallelen Wicklungen dem Schmortest unterzogenen Lackdrahtprobelängen hindurchgeleiteten Heizstromes. Zur genauen
Indikation des Testabschlusses, sind zugleich den Heizdrahtwindungen anliegende Windungen einer zweiten Probelänge des
zu prüfenden Lackdrahtes auf einem indikativen Potenital gehalten, dessen Abfall als unmittelbare Folge der Zerstörung
der Lackisolierung durch die Hitzeeinwirkung zur Anzeige des
Testabschlusses dient, wobei zugleich die Einspeisung des Heizstromes automatisch, z.B. über einen Fehlerstromschalter
ganz abgeschaltet wird.

0054673

Die Vorteile der Erfindung liegen vor allem in der Einfachheit
der Durchführung eines solchen Hitzebeständigkeits- bzw. Hitzedauerbeständigkeitstestes (Schmortest), des geringen hierzu erforderlichen Energiebedarfes sowie der einfachen und gewichtsarmen Ausgestaltung der hierfür eingesetzten Vorrichtung, so
daß diese mit geringem Aufwand herstellbar, handlich zu bedienen und problemlos transportierbar ist.

Die Durchführung des erfindungsgemäßen Schmortestes erfolgt
so, daß auf einen vorzugsweise keramischen Träger in zwei Lagen
übereinander oder mit Abwechslung aufeinander folgenden Windungen zwei Probelängen des zu prüfenden Lackdrahtes aufgewickelt
werden. Sodann wird durch eine dieser parallelen Wicklungen
der Probedrahtlängen ein Heizstrom geringer Spannung geleitet,
während die andere Wicklung auf einer definierten Prüfspannung
z.B. 100 Volt, gehalten wird. Schließlich wird durch den Abfall
der Prüfspannung zufolge der bei Erreichen der Schmortemperatur
bzw. der Hitzedauerstandszeit herbeigeführten Zerstörung der
Isolierung der Heizstrom ganz abgeschaltet, wobei die Schmortemperatur bzw. die Schmorzeit festgestellt wird.

Vorteilhafte Weiterbildungen dieses Verfahrens sind mit den
Unteransprüchen 2 und 3 angegeben, von welchen der Anspruch 2
auf das kontinuierliche Messen des Temperaturanstiegs in den
Probelängen und die entsprechende temperaturabhängige Regelung
der Heizstromzufuhr zwecks Erzielung eines gleichmäßigen linearen Temperaturanstieges gerichtet ist, während der Anspruch 3
eine besonders vorteilhafte Art der temperaturabhängigen Regelung des Heizstromes bei gleichzeitiger optischer Anzeige und
graphischer Darstellung der jeweiligen Temperatur betrifft.

Die Unteransprüche 4 bis 6 sind auf die Ausgestaltung der Vorrichtung zum Prüfen eines Lackdrahtes auf Hitze- bzw. Hitzedauerbeständigkeit gerichtet sind, mit einem Gehäuse, in dem
eine Halterung für wenigstens einen Träger der Probelängen des zu prüfenden Lackdrahtes oder Prüflings sowie eine Heizvorrichtung angeordnet ist. Bei dieser neuen Vorrichtung ist wesentlich, daß statt der eingangs beschriebenen Heizeinrichtung in
Form von in die Gehäusewandung bzw. dessen Boden eingebauter
Heizelemente **ein Heiztrafo mit Stromanschlüssen für den
Prüfling sowie wenigstens eine Prüfspannungs-**
elektrode und ein gemeinsamer Null-Leiter-Anschluß am Gehäuseboden eingebaut sind. Wesentlich ist ferner, daß im Bereich der
Halterung für den Prüfling ein in diesen einführbares Thermoelement beweglich angeordnet ist, dessen hinteres Ende bis zu einem
ebenfalls an der Gehäusewandung angebrachten Temperatur-Regler
geführt ist. Dieser ist zum sequenziellen Betätigen eines Relaisschalters der zum Trafo führenden Stromleitung eingerichtet, wobei der Prüfspannungselektrode ein Relais eines Fehlerstromschalters zugeordnet ist, welcher bei Spannungsverlust des Prüflings die Stromversorgung des Trafos unterbricht.

Weitere Einzelheiten dieser Vorrichtung, deren Vorteile vor
allem in deren einfacher Handhabung und Transportierbarkeit
sowie in dem geringen Energiebedarf bei der Durchführung eines
vorausgehend beschriebenen Schmortestes liegen, sind mit den
Unteransprüchen 5 bis 7 angegeben, von welchen der Anspruch 5
die Ausbildung der Halterung, der Anspruch 6 einen mit dieser
Vorrichtung bevorzugt verwendeten Temperatur-Regler und der
Anspruch 7 die hiermit gekoppelten Anzeige- oder Registriereinrichtungen betrifft.

Nachstehend ist die Erfindung mit weiteren Einzelheiten und
Vorteilen derselben anhand eines in der Zeichnung veranschaulichten Ausführungsbeispieles näher erläutert. In der Zeichnung zeigen:

Fig. 1 einen Längsschnitt durch die Schmortest-Vorrichtung

Fig. 2 in vergrößerter Darstellung einen Ausschnitt aus

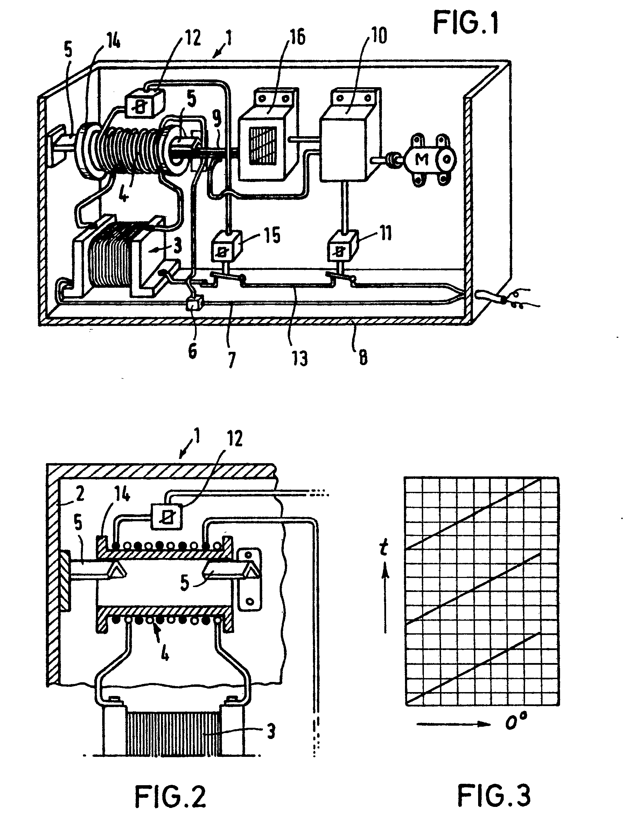

       Fig. 1 und

Fig. 3 ein Zeit-Temperatur-Diagramm einer mit dieser Vorrichtung durchgeführten Schmorprüfung.

Die gezeigte Schmorprüf-Vorrichtung weist ein Gehäuse 1 auf,
an dessen Boden 8   ein Heiztrafo 3 mit Stromanschlüssen für
den Prüfling 4 und wenigstens eine Prüfspannungseleketrode 6,
und in deren Nähe ein gemeinsamer Null-Leiter-Anschluß 7
eingebaut sind, wobei vorzugsweise oberhalb  an der Gehäusewandung 2 eine Halterung 5 für einen Prüfling 4 angebracht
ist. Die Halterung 5 kann jedoch auch als Paar von im Abstand
über dem Gehäuseboden 8 angeordneten keilförmigen Auflagern
für einen überschiebbaren Keramikkörper 14 in Form einer
Zylinderspule ausgebildet sein, auf welche vor deren Auflage
auf die Halterung 5 zwei Probelängen des zu prüfenden Drahtes
derart in nebeneinander liegenden Windungen aufgebracht werden,
daß diese einander berühren, wobei freie Endstücke für deren
Anschluß an den Heiztrafo 3 bzw. an die Prüfspannungselektrode
6 und den gemeinsamen Null-Leiter 7 frei abstehen.

Im Bereich der Halterung 5 für den Prüfling 4 ist ein in diesen
bzw. in die Bohrung der ihn tragenden Zylinderspule einführbares Thermoelement 9 so beweglich angeordnet, daß dessen hinteres Ende bis zu einem ebenfalls an der Gehäusewandung 2 angebrachten Temperatur-Regler 10 führt. Dieser ist zum sequenziellen Betätigen eines Relaisschalters 11, der zum Heiztrafo
3 führenden Zuleitung eingerichtet. Als Regler  wird vorzugsweise ein Proportional-Differenzial-Integral-Regler, Kurzbezeichnung "PDI" eingesetzt, der in Abhängigkeit vom Differenzialwert zwischen einem auf bestimmte Gradientenwerte der Temperatur des Prüflings 14 abgestellten scheibenförmigen Sollwertgeber und den dem Regler 10 vom Thermoelement 9 zugeleiteten Temperatur-Istwerten zur kontinuierlichen Abgabe von Schaltimpulsen an den Traforelaisschalter 11 eingerichtet ist. Um
die jeweilige Temperatur des Prüflings sowie dessen Endtemperatur und/oder eine eingestellte Dauertemperatur kontinuierlich aufschreiben und ablesen zu können, ist der Temperatur-
regler 10 mit einem Anzeige- und Registriergerät 16 gekoppelt,
welches dazu geeignet ist, ein Zeit-Temperatur-Diagramm der
mit dieser Vorrichtung durchgeführten Schmorprüfungen aufzuzeichnen, wie dies in Fig. 3 gezeigt ist.

Der Prüfspannungselektrode 6 ist ein Relais 12 eines Fehlerstromschalters 15 zugeordnet, welcher bei Spannungsverlust des
Prüflings die Stromversorgung des Trafos 3 unterbricht und somit den Schmortest beendet. Dies ist durch einen Abbruch der
Diagrammanzeige am Registriergerät 16 zu erkennen, bei gleichzeitiger Anzeige der bis zum Abbruch des Testes erreichten
Temperatur.

Für die Durchführung einer Hitzedauer-Beständigkeitsprüfung
mit diesem Gerät, wird der Temperatur-Regler 10 zur Aufrechterhaltung einer vorgegebenen Maximalwerttemperatur eingestellt,
bei deren Erreichen er den über die Zuführleitung 13 dem Trafo
3 zugeführten Heizstrom diskontinuierlich in kurzen Zeitintervallen an- und abschaltet, um diese möglichst konstant aufrechtzuerhalten, welche z.B. um 50 $^\circ$C geringer als jene zuvor ermittelte kritische Temperatur ist, bei welcher die Lackisolierung
des Prüflings  sofort zerstört wird. Sodann wird die Zeitdauer
bis zur Zerstörung der Lackdrahtisolierung durch langzeitige
Hitzeeinwirkung gemessen und hieraus die Hitze-Dauerbeständigkeit des Prüflings bestimmt. Es versteht sich, daß dieser Test
längere Zeit in Anspruch nimmt, als ein einfacher Schmortest,
daß jedoch mit Hilfe der neuen Vorrichtung einfacher und mit
wesentlich geringerem Energieaufwand als bisher die Hitzebeständigkeit auch von mit Isolierlack getränkten Systemen, z.B. mit
einem lackisolierten Draht bewickelte und mit einem Tränkmittel
wie Tränklack oder Tränkharz getränkten Spulenwickel, zu prüfen
ist.
Zur üblichen Prüfung werden auf einem Keramikkörper zwei parallele Drähte in Lagen übereinander oder mit abwechselnden Windungen nebeneinander so gewickelt, daß zwei galvanisch getrennte Wicklungen entstehen. Die eine dieser Wicklungen wird sodann
mit einer bestimmten Stromdichte, entsprechend dem Drahtdurchmesser zum Aufheizen der Probelänge des auf seine maximale
Hitzebeständigkeit zu prüfenden Lackdrahtes benutzt, während
die andere Prüfwicklung mit einer definierten Prüfspannung
z.B. 100 Volt beaufschlagt ist, wobei die Heizwicklung das
Gegenpotenzial bildet.

Der Prüfwickel 4 wird hierbei mit einem Temperaturanstieg von beispielsweise 20 $^{o}$C pro Minute aufgeheizt. Die Temperaturüberwachung und -Steuerung übernimmt der entsprechend programmierte Temperatur-Regler 10, der über sein Thermoelement 9 den Temperaturanstieg überwacht und dementsprechend auf den Relaisschalter 11 für den Heizstrom einwirkt, um diesen vorübergehend abzuschalten, und somit den gewünschten linearen Temperaturanstieg zu bewirken. Das Anzeige- und Registriergerät 16, welches mit dem Temperatur-Regler 10 gekoppelt ist, kann durch Impulse des gleichen Thermoelementes 9 oder auch durch ein zweites im Prüfwickel 4 bzw. dessen Keramikträger 14 angeordnetes Thermoelement in Verbindung stehen, mit dem Vorteil einer unabhängigen Überwachung der Funktion des Temperatur-Reglers. Die Hitzebeständigkeitsprüfung ist dann beendet, wenn zwischen den parallelen Windungen ein Fehlerstrom von beispielsweise 500 mmA fließt, welcher die gänzliche Abschaltung des Heizstromes in der Primärwicklung des Trafos 3 durch die Betätigung des Fehlerstromschalters 15 in der zum Trafo führenden Stromleitung 13 bewirkt. Zugleich wird die bei dem betreffenden Test erreichte höchste Temperatur am Anzeige- und Registriergerät 16 aufgezeichnet und läßt sich so ablesen, sowie auch bei späteren vergleichenden Qualitätsprüfungen sowie als Dokumentation der Lackqualität heranziehen.

Fl 4592                                    1                         19.12.80

Ansprüche:

1. Verfahren zum Prüfen eines Lackdrahtes auf Hitze- bzw.
   Hitzedauer-Beständigkeit durch Erhitzen des lackisolierten
   Drahtes bis zum Verschmoren der Isolierung und Feststellung der hierbei erreichten Temperatur bzw. Zeitdauer,
   d a d u r c h   g e k e n n z e i c h n e t ,  daß
   a) auf einen vorzugsweise keramischen Träger in zwei Lagen
      übereinander oder mit abwechselnd aufeinander folgenden
      Windungen zwei Probelängen des zu prüfenden Lackdrahtes
      aufgewickelt werden,
   b) durch eine dieser parallelen Wicklungen der Probedrahtlängen ein Heizstrom geringer Spannung geleitet, während
      die andere Wicklung auf einer definierten Prüfspannung,
      z.B. 100 V, gehalten wird,
   c) durch Abfall der Prüfspannung zufolge der bei Erreichen
      der Schmortemperatur bzw. Hitze-Dauerstandszeit herbeigeführten Zerstörung der Isolierung der Heizstrom ganz
      abgeschaltet, und die Schmor-Temperatur bzw. -Zeit festgestellt wird.

2. Verfahren nach Anspruch 1, d a d u r c h   g e k e n n -
   z e i c h n e t ,  daß der Temperatur-Anstieg innerhalb der
   beiden Wicklungen kontinuierlich gemessen, und der Heizstrom in Abhängigkeit von den jeweils ermittelten Temperatur-
   Istwerten automatisch an- und abgeschaltet wird, wodurch in

0054673

den Probelängen des Lackdrahtes ein gleichmäßiger linearer
Temperaturanstieg mit konstantem Gradienten herbeigeführt
wird.

3. Verfahren nach Anspruch 1 oder 2, d a d u r c h   g e -
k e n n z e i c h n e t, daß die Istwerte der Temperatur
des Prüfwickels optisch angezeigt und graphisch registriert,
sowie auf einen automatischen Temperatur-Regler übertragen
werden, welcher den Heizstrom in dem jeweiligen Temperaturanstieg entsprechenden Zeitintervallen an- und abschaltet.

4. Vorrichtung nach einem der Ansprüche 1 bis 3, mit einem Gehäuse, in dem eine Halterung für wenigstens einen Träger
der Probelängen des zu prüfenden Lackdrahtes oder Prüflings
sowie eine Heizeinrichtung angeordnet ist, d a d u r c h
g e k e n n z e i c h n e t, daß
   a) statt der Heizeinrichtung in der Gehäusewandung (2) ein
      Heiztrafo (3) mit Stromanschlüssen für den Prüfling (4)
      sowie wenigstens eine Prüfspannungselektrode (6) und
      ein gemeinsamer Null-Leiter-Anschluß (7) am Gehäuseboden
      (8) eingebaut sind,
   b) im Bereich der Halterung (5) für den Prüfling (4) ein
      in diesen einführbares Thermoelement (9) beweglich angeordnet ist, dessen hinteres Ende bis zu einem ebenfalls
      an der Gehäusewandung (2) angebrachten Temperatur-Regler
      (10) geführt ist, welcher zum sequentiellen Betätigen
      eines Relaisschalters (11) der zum Trafo (3) führenden
      Stromleitung (13) eingerichtet ist, und
   c) der Prüfspannungselektrode (6) ein Relais (12) eines
      Fehlerstromschalters (15) zugeordnet ist, welcher bei
      Spannungsverlust des Prüflings (4) die Stromversorgung
      des Trafos (3) unterbricht.

5. Vorrichtung nach Anspruch 4, d a d u r c h   g e k e n n -
z e i c h n e t, daß die Halterung (5) als Paar von im Abstand über dem Gehäuseboden (8) angeordneten keilförmigen
Auflagern für einen überschiebbaren Keramikkörper (14) in
Form einer Zylinderspule ausgebildet ist.

Fl 4592 — 3 — 19.12.80

6. Vorrichtung nach Anspruch 4 oder 5, d a d u r c h
   g e k e n n z e i c h n e t , daß der Temperatur-Regler
   (10) ein Proportional-Differential-Integral-Regler (PID)
   ist, der in Abhängigkeit vom Differentialwert zwischen
   einem auf bestimmte Gradientenwerte der Temperatur des
   Prüflings (14) abgestellten scheibenförmigen Sollwertgeber
   und den dem Regler (10) vom Thermoelement (9) zugeleiteten
   Temperatur-Istwerten zur kontinuierlichen Abgabe von Schaltimpulsen an den Trafo-Relaisschalter (11) eingerichtet ist.

7. Vorrichtung nach einem der Ansprüche 4 bis 6, d a d u r c h
   g e k e n n z e i c h n e t , daß der Temperatur-Regler (10)
   mit einem Anzeige- und Registriergerät (16) gekoppelt ist.

FIG.1

FIG.2

FIG.3

Europäisches
Patentamt

**EUROPÄISCHER RECHERCHENBERICHT**

| | **EINSCHLÄGIGE DOKUMENTE** | | KLASSIFIKATION DER ANMELDUNG (Int. Cl.) |
|---|---|---|---|
| Kategorie | Kennzeichnung des Dokuments mit Angabe, soweit erforderlich, der maßgeblichen Teile | betrifft Anspruch | |
| A | DE - A - 1 949 780 (VEREINIGTE DRAHT- UND KABELWERKE A.G.)<br><br>* Seite 1, Zeile 4 - Seite 3, Zeile 22 * | 1 | G 01 N 25/04 33/26<br>G 01 R 31/12 |
| A | DE - C - 526 969 (FELTEN & GUILLEAUME CARLSWERK A.G.)<br><br>* Zeilen 29-66; Figuren * | 1 | |
| A | ELEKTROTECHNIK UND MASCHINENBAU, Band 95, Nr. 4, April 1978, Seiten 193-198<br>Wien, AT.<br>J. MOELLER: "Zuverlässigkeit von Messwandlern"<br><br>* Seite 196, rechte Spalte, Zeile 21 - Seite 197, linke Spalte, Zeile 27; Figur 7 * | 1 | RECHERCHIERTE SACHGEBIETE (Int Cl.)<br><br>G 01 N 25/00 33/00<br>G 01 R 31/00 |
| A | ELECTRICAL WORLD, Band 172, Nr. 4 28. Juli 1969, Seite 40<br>New York, U.S.A.<br>J.G. VALDES: "Hv cable load cycle tests optimized"<br><br>* Insgesamt * | 1 | |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 23-03-1982 | ANTHONY |

EPA form 1503.1 06.78